# EUROPEAN PATENT SPECIFICATION

(11) **EP 1 138 785 B1**
(45) Date of publication and mention of the grant of the patent: **21.10.2009**
(21) Application number: 01108170.0
(22) Date of filing: 30.03.2001
(51) Int. Cl.: C12Q 1/68

(54) **Nucleic acid fragment primer or probe, and method of detecting polyhydroxyalkanoate synthesizing microorganism by using the same**
Nukleinsäurefragment-Primer oder -Sonde und diese verwendendes Verfahren zum Nachweis von Polyhydroxyalkanoat-produzierenden Mikroorganismen die diese verwendet
Fragment d'acide nucléique, amorce ou sonde et procédé les utilisant pour la détection des microorganismes synthétisant le polyhydroxyalkanoate

(30) Priority: 30.03.2000 JP 2000095008
(43) Date of publication of application: 04.10.2001
(73) Proprietor: CANON KABUSHIKI KAISHA, Tokyo (JP)
(72) Inventor: Yano, Tetsuya, Canon Kabushiki Kaisha, Tokyo (JP); Imamura, Takeshi, Canon Kabushiki Kaisha, Tokyo (JP); Suda, Sakae, Canon Kabushiki Kaisha, Tokyo (JP); Honma, Tsutomu, Canon Kabushiki Kaisha, Tokyo (JP)
(74) Representative: Weser, Wolfgang

(56) References cited:
- EP-A- 0 659 765
- EP-A- 0 844 305
- WO-A-98/33893
- JP-T- 06 279 492
- US-A- 5 474 917
- US-A- 5 500 341
- US-A- 5 597 710
- US-A- 5 639 612
- US-A- 5 750 848
- DATABASE EBI [Online] 3 March 2000 (2000-03-03) MARRA M. ET AL.: "The WashU-HHMI Mouse EST project" Database accession no. AA921165 XP002266259
- LOPEZ NANCY I ET AL: "Detection of reserve polymer synthesis genes in natural bacterial populations." FEMS MICROBIOLOGY ECOLOGY, vol. 22, no. 2, 1997, pages 129-136, XP001155332 ISSN: 0168-6496
- PEOPLES O P ET AL: "POLY-BETA-HYDROXYBUTYRATE BIOSYNTHESIS IN ALCALIGENES EUTROPHUS H16CHARACTERIZATION OF THE GENES ENCODING BETA--KETOTHIOLASE AND ACETOACETYL-COA REDUCTASE" JOURNAL OF BIOLOGICAL CHEMISTRY, AMERICAN SOCIETY OF BIOLOGICAL CHEMISTS, BALTIMORE, MD, US, vol. 264, no. 26, 16 September 1989 (1989-09-16), pages 15293-15297, XP002934260 ISSN: 0021-9258
- HUISMAN ET AL: "Metabolism of poly(3-hydroxyalkanoates) (PHAs) by Pseudomonas oleovorans" JOURNAL OF BIOLOGICAL CHEMISTRY, AMERICAN SOCIETY OF BIOLOGICAL CHEMISTS, BALTIMORE, MD, US, vol. 266, no. 4, 5 February 1991 (1991-02-05), pages 2191-2198, XP002087431 ISSN: 0021-9258
- TIMM ET AL: "Cloning and molecular analysis of the poly(3-hydroxyalkanoic acid) gene locu of Pseudomonas aeruginosa PAO1" EUROPEAN JOURNAL OF BIOCHEMISTRY, BERLIN, DE, vol. 1, no. 209, 1 October 1992 (1992-10-01), pages 15-30, XP002077729 ISSN: 0014-2956
- MATSUSAKI HIROMI ET AL: "Cloning and molecular analysis of the poly(3-hydroxybutyrate) and poly(3-hydroxybutyrate-co-3-hydroxyalkanoa te) biosynthesis genes in Pseudomonas sp. strain 61-3" JOURNAL OF BACTERIOLOGY, WASHINGTON, DC, US, vol. 180, no. 24, December 1998 (1998-12), pages 6459-6467, XP002167124 ISSN: 0021-9193
- SHEU DER-SHYAN ET AL: "Rapid detection of polyhydroxyalkanoate-accumulating bacteria isolated from the environment by colony PCR." MICROBIOLOGY (READING), vol. 146, no. 8, August 2000 (2000-08), pages 2019-2025, XP002255569 ISSN: 1350-0872
- SOLAIMAN D K Y ET AL: "Rapid and specific identification of medium-chain-length polyhydroxyalkanoate synthase gene by polymerase chain reaction." APPLIED MICROBIOLOGY AND BIOTECHNOLOGY, vol. 53, no. 6, June 2000 (2000-06), pages 690-694, XP002255570 ISSN: 0175-7598
- SCHEMBRI M A ET AL: "CLONING AND ANLYSIS OF THE POLYHYDROXYALKANOIC ACID SYNTHASE GENE FROM AN ACINETOBACTER SP.: EVIDENCE THAT THE GENE IS BOTH PLASMID AND CHROMOSOMALLY LOCATED" FEMS MICROBIOLOGY LETTERS, AMSTERDAM, NL, vol. 118, no. 1/2, 1 May 1994 (1994-05-01), pages 145-152, XP000674914 ISSN: 0378-1097

## Description

### BACKGROUND OF THE INVENTION

### Field of the invention

The present invention relates to a nucleic acid fragment capable of hybridizing to a polyhydroxyalkanoate, (PHA) synthetase gene DNA derived from a microorganism, a method of detecting a PHA synthetase gene DNA derived from a microorganism or determining a base sequence of a PHA synthetase gene DNA by utilizing the nucleic acid fragment as a primer or probe, as well as a method of detecting or screening a microorganism having PHA synthesizing ability by means of the detection of the above described PHA synthetase gene DNA derived from a microorganism. Related Background Art

There has been many reports about a microorganism having PHA synthesizing ability that produces poly-3-hydroxybutyric acid (PHB) or other PHAs and accumulate them intracellularly ("Biodegradable Plastic Handbook", edited by Biodegradable Plastic Research Society, N.T.S. Co. Ltd., p. 178-197). Such microorganism-produced polymers such as PHA, like a conventional chemically synthesized plastics, can be utilized in producing various products by melt processing, etc. In addition, such microorganism-produced polymers such as PHA, have the advantage of being able to be completely degraded in nature by organisms, since they are biodegradable. Therefore, as opposed to numerous synthetic polymer compounds having been previously used, when scraped, they do not remain in natural environment and cause no environmental pollution. Furthermore, most microorganism-produced PHAs have an excellent biocompatibility and are expected to be applicable to medical soft members, etc.

Such microorganism-produced PHAs have been reported to diversely vary in their compositions and structures depending on the class of microorganisms producing PHAs, and medium composition, incubation condition, etc., used during microorganism culture. To date, for the purpose of improving the properties of PHAs, a method of controlling the composition and structure of PHAs by utilizing the above described means have been investigated.

For example, Alcaligenes eutropus H16 strain (ATCC No. 17699) and its mutants have been reported to produce a copolymer of 3-hydroxybutyric acid (3HB) and 3-hydroxyvaleric acid (3HV) having various composition ratio by varying carbon sources during culture (Japanese Patent Application Laid-Open Nos. 6-15604, 7-14352, 8-19227, etc.) .

Japanese Patent No. 2642937 discloses that Pseudomonas oleovorans strain (ATCC No. 29347), in case where a no cyclic aliphatic hydrocarbons is given as a carbon source, produces PHAs having a monomer unit of 3-hydroxyalkanonate with 6 to 12 carbon atoms.

Japanese Patent Application Laid-Open No. 5-74492 discloses the method comprising contacting a microorganism including Methylobacterium sp., Paracoccus sp., Alcaligenes sp., and Pseudomonas sp. with a primary alcohol with 3 to 7 carbon atoms, thereby allowing them to produce a copolymer of 3HB and 3HV.

Japanese Patent Application Laid-Open No. 5-93049 and 7-265065 disclose that Aeromonas caviae can be cultured by using oleic acid and olive oil as a carbon source to produce a two-component copolymer of 3HB and 3-hydroxyhexanoic acid (3HHx).

Japanese Patent Application Laid-Open No. 9-191893 discloses that Comamonas acidovorans IFO13852 strain can be cultured by using gluconic acid and 1,4-butanediol as a carbon source to produce a polyester having monomer units of 3HB and 4-hydroxybutyric acid.

Furthermore, a certain microorganism has been reported to produce PHA into which various substituents such as, for example, unsaturated hydrocarbon, ester group, aryl group (aromatic ring group), cyano group, halogenated hydrocarbon, epoxide, etc. are introduced, and an attempt to improve the physical properties of microorganism-produced PHAs by means of such a technique is made. For example, it has been reported in Makromol. Chem., 191, 1957-1965, 1990, Macromolecules, 24, 5256-5260, 1991, Chirality, 3, 492-494, 1991, etc., that Pseudomonas oleovorans produces PHAs comprising of as a monomer unit 3-hydroxy-5-phenylvaleric acid (3HPV), and variations in polymer properties probably due to the presence of 3HPV were observed.

As described above, many microorganisms have been reported to produce poly-3-hydroxybutyric acid (PHB) or other PHAs and accumulate them intracellularly; however, the properties of microorganism-produced PHAs are highly attributable to the diversity of PHA synthesizing microorganisms. Thus, it becomes necessary to detect various PHA synthesizing microorganisms that show the diversity in PHA synthesizing ability, and to efficiently screen a strain that show the intended PHA synthesizing ability from a number of PHA synthesizing microorganisms detected above.

In this case, several methods have been previously utilized as means for detecting or screening a PHA synthesizing microorganism. A variety of separation culture processes have been used as means for directly verifying PHA synthesizing ability. In general, selective culture using the special medium, for example, the medium supplemented only with the specific substrate, is frequently performed. Such selective culture processes have been intensively used because of their simplicity, but only a strain having the intended PHA synthesizing ability cannot always selected. Thus, as a method of examining the presence or absence of PHA synthesis in separation culture processes, for example, the method where PHA is stained with Sudan black B (Archives of Biotechnology, 71, 283, 1970), the method where the PHA accumulation is examined by a phase contrast microscope, etc., have been employed. However, there is a possibility of the presence of other strains that are able to be stained with Sudan black B, in addition to a strain that shows PHA synthesizing ability, thus only stainability cannot be strictly regarded as a indicator.

Accordingly, with regard to an individual strain selected by Sudan black B staining, further detailed examination of its morphological or biochemical appearance will be required. The detection and selection of the intended strain based on its morphological or biochemical appearance require a lot of skill and experience, and the technique itself comprises of complicated procedures, and is time-consuming. In this way, the selection of strains based on Sudan black B staining involves various practical problems. Similarly, with respect to the method where PHA accumulation is examined by a phase contrast microscope, the determination of PHA accumulation require a lot of skill and experience, and the facts that the accuracy is insufficient and the technique is complicated are practically serious problem.

As an alternative approach to the above method of determining PHA synthesis per se and detecting the presence or absence of PHA synthesizing ability, a method of detecting the presence or absence of a PHA synthetase gene involved in PHA synthesis may be conceivable. Thus, a method of detecting a base sequence of a nucleic acid specific to a PHA synthetase gene by utilizing a oligonucleotide primer or probe having a complementary base sequence thereof to select only a strain that have the PHA synthetase gene may be also conceivable.

For a PHA synthetase gene, in several strains, their base sequences have been ascertained and reported (Peoples, O. P. and Sinskey, J., J. Biol. Chem., 264, 15293 (1989); Huisman, G.W. et al., J. Biol. Chem., 266, 2191 (1991); Pieper, U. et al., FEMS Microbiol. Lett., 96, 73 (1992); Timm, A. and Steinbuchel, A., Eur. J. Biochem., 209, 15 (1992); Matsusaki, H. et al., J. Bacteriol., 180, 6459 (1998)). In addition, as an example of selecting a region with a high degree of conservation to design a oligonucleotide with reference to these known base sequences, the sequence reported by Timm, A. and Steinbuchel, A., Eur. J. Biochem., 209, 15 (1992) can be shown.

As described above, in order to efficiently detect a PHA synthesizing microorganism and screen a strain having the intended PHA synthesizing ability, it is necessary to know the presence, predominance and growing state of the microorganism in soil. In this case, a method of specifically detecting and determining a microorganism is required, but conventional means for detecting, such as the culture with selective medium by utilizing Sudan black staining, the determination of PHA synthesis by utilizing a phase contrast microscope, have practically many problems in terms of specificity, sensitivity, convenience, and time required for detecting and screening. Particularly, in order to determine with an adequate accuracy, a lot of skill and experience are required, thereby such techniques seemed to be not always suitable for means for efficiently detecting and screening a PHA synthesizing microorganism.

On the other hand, a method of detecting the presence or absence of a PHA synthetase gene is expected to be powerful means with high accuracy. Although a probe or primer for the detection of a PHA synthetase gene is proposed, the selection of a probe or primer selective toward a PHA synthesizing microorganism and common to a wide range of PHA synthesizing microorganisms still exists as the most difficult problem, since it is used as means for detecting and screening a targeted PHA synthesizing microorganism.

Lopez N.I. et al., FEMS Microbiology Ecology 22:129-136, (1997) discloses detection of reserve polymer synthesis genes in natural bacterial populations, wherein screening of indigenous bacteria for the presence of genetic information for the biosynthesis of storage polymers was performed in river water samples using PCR amplification combined with Southern blotting.

Peoples O. et al., The Journal of Biological Chemistry 264(26): 15293-15297, (1989) discloses poly-β-hydroxybutyrate biosynthesis in Alcaligenes eutrophus H16, wherein the poly-β-hydroxybutyrate biosynthetic thiolase gene form Zoogloea ramigera was used as a hybridization probe to screen restriction digests of Alcaligenes eutrophus H16 DNA.

Huisman G. et al., The Journal of Biological Chemistry 266(4): 2191-2198, (February 1991) discloses metabolism of poly (3-hydroxyalkanoate) (PHAs) by Pseudomonas oleovorans, wherein Pseudomonas oleovorans accumulates (3-hydroxyalkanoate) (PHAs) after growth on medium chain length hydrocarbons.

### SUMMARY OF THE INVENTION

The present invention can solve the above described problems and is intended to provide means for detecting the presence and predominance of a PHA synthesizing microorganism in a rapid, convenient and specific, as well as, highly sensitive way, i.e. a nucleic acid fragment that can be utilized as a probe or primer for the detection of a PHA synthetase gene, is highly selective toward a PHA synthesizing microorganism, and can be commonly used among a wide range of PHA synthesizing microorganisms, as well as a method of detecting and screening a PHA synthetase gene by utilizing the above described nucleic acid fragment as a probe or primer. In addition, the present invention is also intended to provide a method of detecting the above described PHA synthetase gene, followed by determining the base sequence of the PHA synthetase gene by using the above described nucleic acid fragment as a primer.

The above objects are achieved by the nucleic acid fragment according to claim 1, the primer or probe according to claim 2, and the method of detecting a PHA synthesizing microorganism according to claim 8. The other claims relate to further developments.

In the subsequent description, the invention as claimed relates to SEQ ID NO 5, and the other sequences SEQ ID NOs 1 to 3 and SEQ ID NOs 6 to 9 in connection therewith. However, said other sequences as such are outside the scope of the invention as claimed.

In order to solve the above described problems, the present inventors have studied the selection of more suitable base sequence for probe or primer for the detection of a PHA synthetase gene, compared to previously proposed base sequences. The present inventors have also studied the selection of the base sequence that can be newly utilized as a probe or primer such that the detection of the PHA synthetase gene permits the presence of a PHA synthetase gene to be detectable among wider range of PHA synthesizing microorganisms in case of isolating a novel microorganism having PHA synthesizing ability from soil. As a result, it was found that, with regard to several kinds of base sequences selected, DNA fragments consisting of these base sequences or complementary base sequences thereof can hybridize to a PHA synthetase gene present in chromosomal DNA of a novel microorganism that shows PHA synthesizing ability with a high selectivity, and can be used as a probe in a variety of hybridization methods or as a primer in the collection of PCR polymerase chain reaction amplification products from. The present inventors have found that either of these techniques can be used to detect a PHA synthetase gene with much higher accuracy, thereby becoming an effective means for detecting a PHA synthesizing microorganism, and have completed the present study.

Thus, a nucleic acid fragment is artificially prepared, having a base sequence that can be utilized as a probe or primer in order to detect a novel microorganism having PHA synthesizing ability and a PHA synthetase gene which the microorganism possesses. In particular, such nucleic acid fragment can be selected from any of base sequences shown in SEQ ID NO: 1 to 9, or complementary base sequences thereof, or modified sequences subjected to a mutation based on these base sequences. When used as a prove or primer, the nucleic acid fragment should be in the form of the nucleic acid fragment that can be utilized as a primer or probe consisting the nucleic acid fragment having the above described specific base sequence, or the nucleic acid fragment consisting of partial sequence in the above base sequence.

In addition, such nucleic acid fragment may have a few mutation so far as its hybridization properties is maintained and the substantial homology of base sequence is kept, for example, can be a nucleic acid fragment with modified sequence subjected to a nonessential mutation based on base sequences shown in SEQ ID NO: 1 to 9 or complementary base sequences thereof, such as partial deletion of base sequence, addition of extra base or base sequence, or substitution of base in base or partial sequence with other base or base sequence, or combination thereof.

The primer comprises a nucleic acid fragment that can be utilized as a primer consisting of the above described base sequence, into which a marker bound onto the molecule of the above described nucleic acid fragment, and/or a moiety capable of binding to a solid-phase carrier may be introduced as an additional modification. Similarly, the probe of the present invention is a probe comprising a nucleic acid fragment that can be utilized as a probe consisting of the above described base sequence, into which a marker bound onto the molecule of the above described nucleic acid fragment, and/or a moiety capable of binding to a solid-phase carrier may be introduced as an additional modification.

The primer can be utilized as, for example, a primer pair in PCR amplification, and, in this case, should be a primer composition comprising a combination of two kinds of nucleic acid fragments with a substantial difference in base sequence, where at least one of the above described two kinds of nucleic acid fragments is the nucleic acid fragment for primer , and
a marker on the molecule, and/or a moiety capable of binding to a solid-phase carrier may be introduced into each molecule of two kinds of nucleic acid fragments.

The primer can be utilized not only as the above described primer pair in PCR amplification, but, for example, as a primer for the preparation of cDNA corresponding to mRNA, and in any uses, the nucleic acid fragment for primer with the above described modified base sequence can be also utilized. In case of utilizing the nucleic acid fragment for primer with the modified base sequence, the primer may be used **characterized in that** the above described base sequence of the nucleic acid fragment for primer to be used is the modified base sequence subjected to a mutation, such as partial deletion of base sequence, addition of extra base or base sequence, or substitution of base or partial sequence in base sequence with other base or base sequence, or combination thereof, based on base sequences shown in SEQ ID NO: 1 to 9 or complementary base sequences thereof.

The primer or probe may be subjected to an additional modification, as mentioned above. Therefore, in this case, the primer or probe **characterized in that** it comprises at least one kind of nucleic acid fragment subjected to an additional modification, and the additional modification in the above described one kind of nucleic acid fragment is the introduction of a marker, or moiety capable of binding to a solid-state carrier into the side of 5'-terminal of the nucleic acid fragment may be used.

For example, the primer or probe is preferably used wherein , as an additional modification, the marker or moiety capable of bounding onto a solid-state carrier that is introduced into the molecule is any of biotin residue, 2,4-dinitrophenyl group, digoxigenin residue.

Furthermore, in a method for detecting a PHA synthesizing microorganism may be at least one kind of nucleic acid fragment is any of the nucleic acid fragment with the above described base sequence the morphology of a nucleic acid fragment that can be utilized as a primer or probe, or the nucleic acid fragment with the modified sequence subjected to a mutation, such as partial deletion of base sequence, addition of extra base or base sequence, or substitution of base or partial sequence in base sequence with other base or base sequence, or combination thereof, is used as a probe. Alternatively, in a method for detecting a PHA synthesizing microorganism may be , at least one kind of nucleic acid fragment is any of the nucleic acid fragment with the above described base sequence the morphology of a nucleic acid fragment that can be utilized as a primer or probe, or the nucleic acid fragment with the modified sequence subjected to a mutation, such as partial deletion of base sequence, addition of extra base or base sequence, or substitution of base or partial sequence in base sequence with other base or base sequence, or combination thereof, is used as a primer

For example, the method for detecting a PHA synthesizing microorganism may use the above described primer , and may comprise the following four steps:
(1) preparing a sample in which the presence or absence of a PHA synthesizing microorganism is to be detected.
(2) performing a lysis treatment of cells in the sample, if necessary,
(3) a step for adding the above described primer to the sample and performing elongation reaction of the primer, and
(4) a step for performing a detecting operation of elongation reaction products obtained from step (3), or
   the above described steps (1), (3), and (4), as well as step (2), if necessary. Notably, it is more preferred to perform a method of detecting a PHA synthesizing microorganism **characterized in that** a primer consisting of combination of the above described two kinds of nucleic acid fragments is used. Thus, it is much preferred to perform a method of detecting a PHA synthesizing microorganism **characterized in that** the elongation reaction of primer in step (3) is conducted by polymerase chain reaction.
   The nucleic acid fragment of claim 1 on 5 or can be utilized as a primer or probe to specifically detect a PHA synthesizing microorganism. In addition, the method of detecting a PHA synthesizing microorganism with use of the primer or probe of the present invention will be an excellent detection method in terms of its detection sensitivity, specificity, simplicity of procedures and rapidity. Such a high degree of efficiency in the detection of a PHA synthesizing microorganism will contribute greatly to the development of PHAs produced by utilizing a PHA synthesizing microorganism, for example, the research and development in the field of biodegradable plastic, etc.

### DETAILED DESCRIPTION OF THE PREFERRED EMBODIMENTS

Nucleic acid fragments an claimed retain substantially base sequences designated as SEQ ID NO 5, and optionally SEQ ID NOs: 1 to 4 or the complementary base sequences, and have characteristics capable of hybridizing in high selectivity with genes of a PHA synthesizing enzyme present in the chromosome DNA for different microorganisms showing PHA synthesizing ability. Any of the base sequences designated as SEQ ID NOs: 1 to 9 are composed of about 25 bases, namely 23 to 27 bases for the sequence length. The nucleic acid fragments as claimed selectively hybridize with any strand of double-stranded DNA of the gene of the PHA synthesizing enzyme as the single-stranded DNA molecule retaining substantially these base sequences or their complementary base sequences.

The followings will be described more specifically for nucleic acid fragments of the present invention, their using form as probes or primers and detection methods of PHA synthesizing microorganisms using them as the probes or primers.

### <Nucleic acid fragments>

As described above, nucleic acid fragments of the present a description are nucleic acid having base sequences designated as SEQ ID NOs: 1 to 9 or their complementary base sequences, or nucleic acid fragments having substantially the base sequences selected from any of modified sequences where variation has been performed based on these base sequences. In other words, the nucleic acid fragments of the present invention are single-stranded nucleic acid fragments set as the length of 10 to 50 bases for the sequence length according to their use when using them as primers or probes.

Thus, when utilizing them as the primers, any of the base sequences designated as SEQ ID NOs: 1 to 9 are composed of about 25 bases, namely 23 to 27 bases for the sequence length, however, they may be set, for example, as the nucleic acid fragments of 10 bases in the minimum overall length or as the nucleic acid fragments of 10 bases in the minimum overall length having the complementary base sequences, selecting their partial base sequences. On the other hand, when using them as the probes, for example, they may be set as the nucleic acid fragments of 50 bases in the maximum overall length coupling additional base sequences with the base sequences composed of about 25 bases or as the nucleic acid fragments of 50 bases in the maximum overall length coupling additional base sequences with the complementary base sequences composed of about 25 bases. Herein, for the nucleic acid fragments of the present invention, when using only the partial base sequences based on the base sequences designated as SEQ ID NOs: 1 to 9 or their complementary base sequences, it is preferable to select the portion where even only the partial base sequences are specific to PHA synthesizing microorganisms and less homologous to other bacteria.

Or they may have addition, insertion and deletion substituting partial base sequences within the range of retaining hybridization ability substantially against the genes of the PHA synthesizing enzymes based on the base sequences designated as SEQ ID NOs: 1 to 9 or their complementary base sequences. For example, when utilizing the nucleic acid fragments of the present invention as primers used for PCR reaction, they can be manipulated with variation such as substitution of the bases and removal of the terminal sequences has been performed within the range of retaining hybridization ability against the genes of the PHA synthesizing enzymes so as not to cause hybridization between the primers each other. In addition, when double-stranded DNA was formed by the PCR reaction, they can be treated with base sequences to perform manipulations such as addition, insertion and the like so as to contain breakage sequences by restriction enzymes in the parts originated in the nucleic acid fragments of the present invention. Or when being generally used as the mixed primers, substitution of bases can be performed according to the range of codon degeneracy in the range of coincidence with amino acid sequences coded in the base sequences to prepare a mixture of plural types having the analogous base sequences each other.

Herein, modification or addition/insertion of the aforementioned base sequences accompanying the modification may be selected so as not to induce high hybridization ability with genes other than those of the objective PHA synthesizing enzymes. For example, for microorganisms retaining genes of the PHA synthesizing enzymes, they may be selected so as not to become modification possibly to damage selectivity like introduction of base sequences inducing high hybridization ability with genes of other enzyme proteins associated with metabolic reactions of alkanoic acids. Further, for the microorganisms which have metabolic ability of alkanoic acids although the genes of the PHA synthesizing enzymes themselves are not retained, they may be selected so as not to become variation of the base sequences possibly to damage selectivity by introduction of base sequences inducing high hybridization ability with genes of other enzyme proteins associated with metabolic reactions of alkanoic acids.

The nucleic acid fragments , as described above, are the base sequences where, for example, deletion, substitution, addition, etc. of the partial bases or base sequences have been performed and can be prepared as nucleic acid fragments having the length of 10-50 bases according to the use mode and objects as probes or primers. Particularly, in the case of utilizing the nucleic acid fragments as the primers, the elongation reaction of primers is performed using the genes of the PHA synthesizing enzymes as a template, herein it is usually preferable either to avoid variation near the 3'-terminal which has probably significant effect on the elongation reaction or to minimize the base number to be varied even if variation occurs near the 3'-terminal. Accordingly, when introducing the variation such as additional base sequences not having action of complementing or promoting the intrinsic hybridization ability, it is more suitable to be varied near the 5'-terminal.

In addition, for the primers or probes using the nucleic acid fragments of the present invention, the nucleic acid fragments may be the ones composed of the length of the above described 10 to 50 bases and modified by introducing regions capable of coupling with a marker and/or the solid-phase carrier on the DNA molecule. As described below, regions capable of coupling with this marker and/or the solid-phase carrier has the role of detection or fixation of the double-stranded DNA fragments or hybridized DNA complexes using regions capable of coupling with the concerned marker and/or the solid-phase carrier. This additional modification is not particularly limited to the introduced regions as long as it does not damage the hybridization ability of the primers or probes by use of the nucleic acid fragments of the present invention to the genes of PHA synthesizing enzymes.

According to such requirement, the primers with regions capable of coupling with the marker or the solid-phase carrier introduced perform the detection of PHA synthesizing microorganisms by carrying out, for example, elongation reaction of 3'-terminal using the genes of the PHA synthesizing enzymes as a template utilizing these primers. Specifically, while the position at which regions capable of coupling with the marker or the solid-phase carrier can be introduced may be anywhere as long as the elongation reaction of the primers is not interfered, the introduction to the 5'-terminal is preferable if possible. Further, when performing the detection using the probes, the elongation reaction to the 3'-terminal of the probes is not commonly employed. Accordingly, for the position of the probes at which regions capable of coupling with the marker or the solid-phase carrier can be introduced, hydroxyl group portions at the 3'- and 5'-terminals, further the base portion, the phosphodiester portion and the like can be also employed. In addition, it is desirable to introduce to the position where hybridization is not interfered considering the base sequence of the probe itself and its length. For example, it is also desirable to employ the additional base sequence portion except for the portions of the base sequences designated as SEQ ID NOs: 1 to 9 or their complementary base sequences, mainly associated with hybridization in the whole base sequences of the probe as the position for introduction.

Nucleic acid fragments of the present invention are set as the single-stranded DNA when used since they are primarily utilized as the primers or probes, while when preparing, they may be the form of double strand DNA where the single strand DNAs having complementary base sequences each other are coupled. These nucleic acid fragments or their partial base sequences of the present invention can be prepared in fit forms by optional methods. The whole or a part of base sequences may be chemically synthesized according to the base sequences of the nucleic acid fragments, for example, according to the methods of the examples described later. Optionally, it is also possible to use the amplified fragments themselves or their partial fragments cut off from the amplified fragments as probes after amplifying by the PCR method using chemically synthesized primers. Further it is also possible to prepare by cutting off directly the specified region of the genes of PHA synthesizing microorganisms detected once by the nucleic acid fragments of the present invention using the restriction enzymes and the like. Furthermore, it is also possible to perform cloning of these genes with the plasmids such as E. coli followed by growing the bacteria and collecting them to cut off the specified region to be used.

### <Region Capable of Coupling with Labeling Material and Solid-Phase Carrier>

When using the above described nucleic acid fragments as the probes or primers, either radioactive or nonradioactive materials may be used as the above described markers. When using a radioactive material, e.g. the one containing the radioactive isotope in the phosphate portion is appropriate. The nonradioactive marker includes fluorescent materials such as fluorescein derivatives, rhodamine and its derivatives, chemoluminescent materials and delayed fluorescent materials. Further, it is also possible to detect the markers indirectly using the substances which couple specifically with the markers. Such indirectly detectable markers include biotin and hapten. For example, avidin or streptoavidin is used for biotin, while for hapten, the antibody coupling specifically with that is used for detection. As the hapten to be used for labeling, the compounds having the 2, 4-dinitrophenyl group, digoxigenin and the like can be used. Each of such markers can be also introduced into the probes or primers combining single or multiple types if necessary.

The region capable of coupling with the solid-phase carrier is used e.g. when coupling the specific fragment of nucleic acid with the solid-phase carrier specifically such as sandwich hybridization. Herein, any region may be used as long as they can couple selectively with the concerned solid-phase carrier. For example, there are biotin or hapten such as fluorescein, compounds having the 2, 4-dinitrophenyl group and digoxigenin. Each of them can be introduced into the probes or primers using single types or combining multiple types if necessary according to the type of the solid-phase carrier. In addition, the additional modification performed on the nucleic acid fragments, primers or probes of the present invention by introducing the region capable of coupling with the markers and/or solid-phase carrier is not limited to the above described examples.

The detection method for PHA synthesizing microorganisms of the present invention is the method utilizing the primers or probes composed of the above described nucleic acid fragments of the present invention.

### <Detection Method Using Probes>

The detection method for PHA synthesizing microorganisms of the present invention using the probes is characterized by use of at least one type of the probes of the present invention composed of the nucleic acid fragments into which the region capable of coupling with the marker and/or the solid-phase carrier may be introduced wherein the nucleic acid fragments have the above described base sequences. Generally, as the detection method using the probes, there are forms of dot hybridization, southern hybridization and in situ hybridization where hybridization can be performed according to the conventional method using the nucleic acid fragments where the above described labeling has been performed. Also in situ hybridization, the nucleic acid fragments of the present invention can be provided for detection.

Further, the procedure based on the sandwich hybridization has been developed in order to simplify the handling of hybridization so that the detection of PHA synthesizing microorganisms can be performed by applying this procedure using the nucleic acid fragments of the present invention for the fixed probes and the like.

In most cases, the detection is carried out by hybridization with the genes of PHA synthesizing enzymes, however, the detection may be performed by the hybridization with the mRNA transcribed from the genes of PHA synthesizing enzymes or with its cDNA.

### <Detection Method Using Primers>

The detection method for PHA synthesizing microorganisms according to the present invention using the primer is the one characterized using at least one type of the primers of the present invention composed of the nucleic acid fragments into which the region capable of coupling with the markers and/or the solid-phase carrier may be introduced wherein the nucleic acid fragments has the above described base sequences. A more preferable example is the detection method applying the PCR (Polymerase Chain Reaction) method where a very small amount of nucleic acid fragments in the sample are amplified by a gene-amplifying reaction utilizing two different types of primers to be described later. Herein, when using two types of primers, the primer forms include e.g. the one where both two types of primers are not modified at all, the one where the region capable of coupling with the detectable label or the solid-phase carrier is introduced into at least one of the two types of primers, the one where the marker is introduced into one of the two types of primers and a region capable of coupling with the solid-phase carrier is introduced into the another type and the one where the regions capable of coupling with the solid-phase carrier are introduced into both two types of primers.

The detection method for PHA synthesizing microorganisms according to the present invention can be performed as follows using at least one type of the primers of the present invention as described above, preferably using a pair of the primers composed of combination of two types of primers:
(1) a step of preparing the sample in which the presence or absence of a PHA synthesizing microorganism is to be detected,
(2) a step of performing a lysis treatment of cell in the sample if necessary,
(3) a step of adding the above described primer to the sample and performing the elongation reaction of the primer,
(4) a step of performing a detecting operation of the elongation reaction obtained from the step (3), or the method is performed by a series of the steps including the above described processes, (1), (3), (4) and further (2) if necessary.

In other words, this method is performed to detect whether there are base sequences complementary to the primers of the present invention from many kinds of nucleic acid fragments, DNA genes and the like contained in the samples using them as its template and detecting generation of products from the elongation reaction of the primer. Herein, application of the PCR method to the elongation reaction of the primers enable selective amplification of the reaction products to attain higher sensitivity of detection. In addition, the molecular weight (base length) of the amplified products becomes a specific amount leading easier detection.

For detection of the nucleic acid fragments amplified by the elongation reaction of the primers in the process (4), commonly used methods such as electrophoresis and hybridization may be used. Further for genetic amplification reaction, it is possible to use the methods such as ones where different labels are introduced into each primer and after amplification reaction, the reaction products are adsorbed on the solid-phase carrier to be detected selectively.

The above described solid-phase carrier includes those where streptoavidin, antibody or the like capable of capturing the coupling region introduced into the primers are introduced into solid-phase carriers such as polystyrene balls, agarose beads, polyacryl beads, latex, and microtiter well. For example, the carrier with streptoavidin coupled with the solid-phase may be used for capturing the PCR products from the primer with biotin introduced, while the carrier with the antibody to fluorescein and the like coupled with the solid-phase may be used for capturing the products of elongation reaction from the primers with fluorescein and the like introduced. Further, making fine-grains of the solid-phase carrier enables simple judgement through judging whether there is aggregation of the nucleic acid fragments of the objective reaction products or formation of precipitation.

Further, the following procedure has been invented: the elongation reaction is performed using two types of primers where the region capable of coupling with the solid-state carrier is introduced into one of the primers, while the marker is introduced into another primer, then the products of the elongation reaction are made to contact with the solid-phase carrier and the impurities which do not couple with the solid-phase carrier are washed to be removed with appropriate solvents. By use of this procedure, the objective nucleic acid fragments having the region capable of coupling with the solid-phase carrier are fixed on the concerned solid-phase carrier in the form which has the marker to be detected more specifically. For practical detection of the marker of the nucleic acid fragments fixed on these solid-phase carrier, conventional procedures may be used according to the used markers. For example, when the marker is a radioisotope, the radioactivity may be measured directly. When it is biotin or hapten, avidin-enzyme combination or antibody-enzyme combination, respectively, may be used to react with the substrate such as AMPPD to the enzymes of the above described combination to detect the amount of enzymes (enzymatic activity) through coloring or fluorescent measures.

Further, for elongation reaction of the primers, e.g. for enzymes, reaction condition and so on to be used for genetic PCR amplification reaction, the various procedures have been invented. The nucleic acid fragments of the present invention based on the base sequences designated as SEQ ID NOs: 1 to 9 have adequate length and base sequences to be used as the primers for any of those different PCR methods. Accordingly, when any of different PCR methods are applied, detection of PHA synthesizing microorganisms can be carried out using the nucleic acid fragments of the present invention performing additional modification, regulation of the base length, introduction of variation as described above and so on if necessary.

The nucleic acid fragments of the present invention has the portion for choices of plural types of bases at one or plural positions in the base sequence designated as SEQ ID NO: 5 which is the basis of the nucleic acid fragments of the present invention. More over, the base sequences designated as SEQ ID NOs: 1 to 9 compose a group of base sequences having homology each other by combination of the above described choices. The nucleic acid fragments of the present invention have the above described base sequences selected from a group of base sequences having homology each other. When using as the primers or probe, one type of nucleic acid fragment having the base sequence selected from a group having any homology designated as SEQ ID NO: 5 may be used. Alternatively, a mixture of plural nucleic acid fragments having homology each other selected from a group having individual homology may be used. Further, the mixture so called mix primer, that is the one containing all base sequences which compose a group having homology may be also used. That is, the nucleic acid fragments having the base sequences designated as SEQ ID NOs: 1 to 9 include, as described above, all of those of single type, the mixture of plural types having homology each other, and further the whole group which has the base sequences having homology each other composed of combination of the choices.

### Examples

The present invention will be more specifically described through examples as follows. Herein, while the following examples described are each of the best embodiments of the present invention, the technical scope of the present invention is not limited to these examples.

### (Example 1) Evaluation of Primer Specificity-1

The primers of the present invention were proved to show the specificity capable of detecting the PHA synthesizing microorganisms. The example will be shown where detection of partial base sequences of the objective PHA synthesizing enzyme genes using genetic DNAs of PHA synthesizing microorganisms as templates by the PCR method utilizing the primers of the present invention.

Using objective 6 bacteria in total which are the known 2 strains; E. coli JM109 and J1 (FERM BP-5352) not having the PHA synthesizing ability and 4 strains; P. cichorii YN2 (FERM BP-7375), P. cichorii H45 (FERM BP-7374), P. putida P91 (FERM BP-7373) and P. jessenii P161 (FERM BP-7376) having the PHA synthesizing ability, their specificity on detection of the PHA synthesizing microorganisms was verified. Namely, for each of six bacteria, the DNA samples were prepared from each different bacterium using the conventional method to evaluate the specificity of primers by the PCR method.

In this example, PCR-amplified products were obtained using 7 mix primers having the base sequences designated as SEQ ID NOs: 1 to 4 and 6 to 8 which are prepared based on the base sequences designated as SEQ ID NOs: 1 to 9, and 2 mix primers having the complementary base sequences in SEQ ID NOs: 5 and 9. Specifically, the following 7 forward-primers (Amersham-Pharmacia-Biotec Co., Ltd.);
(SEQ ID NO: 1):
   5'-GCCTCKGAAAACACCYTGGGSCT-3'
(SEQ ID NO: 2):
   5'-TGACCGARGCCWTSGCSCCGACC-3'
(SEQ ID NO: 3):
   5'-AGCCTGGCGCGSTTCTGCCTGCGC-3'
(SEQ ID NO: 4):
   5'-GGCGARAASAAGGTCAAYGCCYTSACC-3'
(SEQ ID NO: 6):
   5'-TGCAGGCCTAYCTGRSCTGGCAGAA-3'
(SEQ ID NO: 7):
   5'-CCAGTACRYSCTSAARAAYGGCCTGC-3'
(SEQ ID NO: 8):
   5'-CTGGACTTCTTCAAGCWCAACCCG-3'
   and also the following 2 reverse-primers (Amersham-Pharmacia-Biotec Co., Ltd.) which were entrusted in their syntheses were used;
(complementary strand of SEQ ID NO:5)
   5'-CAGCCACCAGGARTCGGYRTGCTTG-3'
(complementary strand of SEQ ID NO: 9)
   5'-ATGCTCTGSAYRTGYCCGCTGTTGG-3'
   (wherein K = G or T, Y = C or T, S = G or C, R = A or G, W = A or T, B = T, G or C, and V = A, G or C).

As the combination of primers for PCR, combination of 7 types in total was set; the combination of 4 primers where 4 forward-primers having the base sequences designated as the above described SEQ ID NOs: 1 to 4 are combined with the reverse-primers having the complementary strand base sequences designated as the SEQ ID NO: 5, and the combination of 3 primers where 3 forward-primers having the base sequences designated as the above described SEQ ID NOs: 6 to 8 are combined with the reverse-primers having the complementary strand base sequences designated as the SEQ ID NO: 9.

The PCR was performed using the commercially available enzyme system, the kit of AmpliTaq DNA polymerase (Takara Shuzo Co., Ltd.) in the following composition of reaction solution and the condition.

The overall amount of the reaction solution was adjusted to 50 µl by adding 1 µl each of the above described two primers having concentration of 50 pmol/pl, 5 µl of the reaction buffer attached to the enzyme, 5 µl of the dNTP-mixed solution attached to the enzyme, 10 ng of the DNA sample and further water. One unit of AmpliTaq DNA polymerase (Takara Shuzo Co., Ltd.) was added to this solution.

After the reaction solution was heated at 95° C and maintained for 5 min, the reaction condition was set as one cycle of being at 95° C for 20 sec, at 60° C for 30 sec and 72° C for 60 sec, and the reaction of 15 cycles was performed under this condition. Further, the reaction condition was set as one cycle of being at 95° C for 20 sec, at 55° C for 30 sec and 72° C for 60 sec, and the reaction of 20 cycles was performed under this condition, then the solution was further kept at 72° C for 5 min. After completion of the reaction, 2 µl was separately taken from 50 µl of the reaction solution, and the agarose gel electrophoresis and the ethidium bromide staining were performed to detect nucleic acid strands of the amplified products.

As a result, for the known 2 strains; E. coli JM109 and J1 (FERM BP-5352) not having the PHA synthesizing ability, no amplified products were detected at all. On the other hand, only for the PHA synthesizing microorganisms, one clear band was observed in any 7 types of PHA synthesizing microorganisms combined with the above described primers for the PCR as each amplified product. Herein, for the above described all 4 PHA synthesizing microorganisms, the fragment lengths of the PCR amplified products obtained from each of 7 types combined with the PCR primers correspond each other, which were shown in the following Table 1. In other words, since combination of 7 primers in total amplified the corresponding partial base sequences to each other from the PHA synthesizing enzyme genes originated in each of 4 PHA synthesizing microorganisms, the PCR-amplified products had almost the same base pair. From the above results, it was confirmed that any of 9 primers used in this example showed specificity applicable for detection of the PHA synthesizing microorganisms.

**Table 1**

| Base Sequence of Forward-Primer | Base Sequence of Reverse-Primer | PCR-Amplified Fragment Length |
|---|---|---|
| SEQ ID NO: 1 | complementary strand of SEQ ID NO: 5 | about 1.5 kbp |
| SEQ ID NO: 2 | complementary strand of SEQ ID NO: 5 | about 1.2 kbp |
| SEQ ID NO: 3 | complementary strand of SEQ ID NO: 5 | about 0.85 kbp |
| SEQ ID NO: 4 | complementary strand of SEQ ID NO: 5 | about 0.6 kbp |
| SEQ ID NO: 6 | complementary strand of SEQ ID NO: 9 | about 1.2 kbp |
| SEQ ID NO: 7 | complementary strand of SEQ ID NO: 9 | about 0.75 kbp |
| SEQ ID NO: 8 | complementary strand of SEQ ID NO: 9 | about 0.2 kbp |

### (Example 2) Preparation of Primer

As an example of nucleic acid fragments of the present invention, for a primer in which a region capable of coupling with a marker or a solid-phase carrier is introduced and for a primer in which a region capable of coupling with a marker or a solid-phase carrier is not introduced, each of them was prepared by a chemical synthetic method.

First, the nucleic acid fragments without introduction of the region capable of coupling with either the marker or the solid-phase carrier were synthesized as single strand DNA by the Phosphoamidite method in a 0.2 µmol scale using the automatic DNA synthesizer model 381A (Perkin-Elmer). The objective nucleic acid fragments were purified through OPC cartridge (Perkin-Elmer) to remove the mixture such as the raw material.

Further, as an example of the primer in which the region capable of coupling with the marker or the solid-phase carrier is introduced, the primer in which the region capable of coupling the marker or the solid-phase carrier was added to the 5'-terminal of the base sequence was prepared. The oligonucleotide with an amino group introduced at the 5'-terminal was chemically synthesized as an intermediate material beforehand, then the region capable of coupling with the marker or the solid-phase carrier was introduced by use of the amino group at the 5'-terminal using appropriate agents. For example, an example of biotinylation and an example of adding a 2, 4-dinitrophenyl group will be described as follows.

Synthesis of Primer Biotinylated at 5'-Terminal
Oligonucleotide introduced with an amino group at the 5'-terminal (SEQ ID NO: 10):
5'-GCCTCGGAAAACACCTTGGGGCT-3'

After adding the final base (this case is C) by the synthetic reaction using the above described Phosphoamidite method, G having an amino group at the 5'-terminal was added by further adding Amino Link II (Perkin-Elmer) to synthesize the above Formula 1. After completion of the synthesis, the oligonucleotide with the amino group introduced at the 5'-terminal of the intermediate material was purified similarly through the OPC cartridge.

In addition, according to the aforementioned procedure, 6 oligonucleotides with the amino group introduced at the 5'-terminal, as described below, were prepared as intermediate materials, and 7 oligonucleotides in total introduced with the amino group at the 5'-terminal were obtained as intermediate materials.

Oligonucleotide with an amino group introduced at the 5'-terminal (SEQ ID NO: 11):
5'-TGACCGAAGCCATGGCGCCGACC-3'

Oligonucleotide with an amino group introduced at the 5'-terminal (SEQ ID NO: 12):
5'-AGCCTGGCGCGGTTCTGCCTGCGC-3'

Oligonucleotide with an amino group introduced at the 5'-terminal (SEQ ID NO: 13):
5'-GGCGAAAACAAGGTCAACGCCCTGACC-3'

Oligonucleotide with an amino group introduced at the 5'-terminal (SEQ ID NO: 14):
5'-TGCAGGCCTACCTGAGCTGGCAGAA-3'

Oligonucleotide with an amino group introduced at the 5'-terminal (SEQ ID NO: 15):
5'-CCAGTACGCGCTGAAGAACGGCCTGC-3'

Oligonucleotide with an amino group introduced at the 5'-terminal (SEQ ID NO: 16):
5'-CTGGACTTCTTCAAGCACAACCCG-3'

Subsequently, biotinylation to the 5'-terminal was carried out as follows. 10 µl of 1M NaHCO₃ aqueous solution, 30 µl of water and 50 µl of DMF solution of 20 pg/pl biotinyl-N-hydroxysuccinimido ester (BRL) as a biotinylation agent were added to 10 µl of 10. D. aminated oligonucleotide aqueous solution, mixed and allowed to stand at room temperature. After 4 hr, gel filtration was performed with Sephadex G-50 as the carrier, eluted with 50 mM TEAB (triethyl ammonium hydrogen carbonate) buffer (pH 7.5) and the first peak was collected. After drying this eluate to solid, it was dissolved in the TE buffer (pH 8.0). The following 6 types were prepared by biotinylation of the intermediate materials that are oligonucleotides with an amino group introduced at the 5'-terminal.

### Synthesis of Primer Introduced with Dinitrophenyl Group (DNP) at 5'-Terminal

Introduction of a dinitrophenyl group (DNP) at the 5'-terminal was carried out using oligonucleotide with an amino group introduced at the 5'-terminal as the intermediate material similarly to biotin-labeling. 2 types of oligonucleotides with the amino group introduced at each 5'-terminal which are (complementary strand of SEQ ID NO: 17):
5'-CAGCCACCAGGAGTCGGCGTGCTTG-3'
and (complementary strand of SEQ ID NO: 18)
5'-ATGCTCTGGACATGCCCGCTGTTGG-3'

The above compounds were synthesized similarly to the above described biotin-labeling and then purified. To the 180 µl of the purified 2 O.D. aminated oligonucleotide aqueous solution, 20 µl of 1M NaHCO₃ aqueous solution was added, then 100 µl of the reagent which is ethanol solution of 5% (v/v) dinitrofluorobenzene was added and the reaction was carried out by heating at 37° C for 2 hr. Similarly to biotinylated oligonucleotide, purification after the reaction, was performed through gel filtration, dried to solid and dissolved in the TE buffer (pH 8.0).

### (Example 3) Preparation of Probe

As an example of the nucleic acid fragments of the present invention, the probe with the region capable of coupling with the marker or the solid-phase carrier introduced was prepared by the chemical synthetic method.

Oligonucleotide with biotin-label introduced at the 3'-terminal (SEQ ID NO: 13):
5'-GGCGAAAACAAGGTCAACGCCCTGACC-Biotin-3'
was prepared as follows. The oligonucleotide having a specific base sequence was synthesized by the phosphoamidite method using 3'-Biotin-ONCPG column (CLONTECH) where the 3'-terminal was biotin-labeled beforehand on a 0.5 µmol scale and eluted of the column. This nucleotide having the biotin-label at the 3'-terminal was also by the conventional method, purified using an OPC cartridge, dried to solid, then dissolved in the TE buffer (pH 8.0). This nucleic acid fragment has biotin-label introduced at the 3'-terminal and suitable not as a primer but as a probe.

### (Example 4) Evaluation of Primer specificity-2

Primers of the present invention were validated whether they exhibited specificity capable of detecting PHA synthesizing microorganisms. One example will be shown below as the one that the partial base sequences of the objective PHA synthesizing enzyme genes were detected by the PCR method using the primers of the present invention as templates of DNAs of the PHA synthesizing microorganisms.

Using objective 6 bacteria in total being the known 2 strains; E. coli JM109 and J1 (FERM BP-5352) not having the PHA synthesizing ability and 4 strains; P. cichorii YN2 (FERM BP-7375), P. cichorii H45 (FERM BP-7374), P. putida P91 (FERM BP-7373) and P. jessenii P161 (FERM BP-7376) having the PHA synthesizing ability, their specificity was verified on detection of the PHA synthesizing microorganisms. Namely, for each of six bacteria, the DNA samples were prepared by each different bacterium using the conventional method to evaluate the specificity of primers by the PCR method.

In the present example, the primers are 9 primers prepared in the example 2, specifically the following 7 forward-primers biotinylated at the 5'-terminal were used;
oligonucleotide biotinylated at the 5'-terminal (SEQ ID NO: 10):
5'-Biotin-GCCTCGGAAAACACCTTGGGGCT-3'
oligonucleotide biotinylated at the 5'-terminal (SEQ ID NO: 11):
5'-Biotin-TGACCGAAGCCATGGCGCCGACC-3'
oligonucleotide biotinylated at the 5'-terminal (SEQ ID NO: 12):
5'-Biotin-AGCCTGGCGCGGTTCTGCCTGCGC-3'
oligonucleotide biotinylated at the 5'-terminal (SEQ ID NO: 13):
5'-Biotin-GGCGAAAACAAGGTCAACGCCCTGACC-3'
oligonucleotide biotinylated at the 5'-terminal (SEQ ID NO: 14):
5'-Biotin-TGCAGGCCTACCTGAGCTGGCAGAA-3'
oligonucleotide biotinylated at the 5'-terminal (SEQ ID NO: 15):
5'-Biotin-CCAGTACGCGCTGAAGAACGGCCTGC-3'
oligonucleotide biotinylated at the 5'-terminal (SEQ ID NO: 16):
5'-Biotin-CTGGACTTCTTCAAGCACAACCCG-3'
and the following 2 reverse-primers having a dinitrophenyl group (DNP) introduced at the 5'-terminal;
oligonucleotide having DNP introduced at the 5'-terminal (complementary strand of SEQ ID NO: 17):
5'-DNP-CAGCCACCAGGAGTCGGCGTGCTTG-3'
oligonucleotide having DNP introduced at the 5'-terminal (complementary strand of SEQ ID NO: 18):
5'-DNP-ATGCTCTGGACATGCCCGCTGTTGG-3'
were used.

Combination of primers for the PCR is that of 4 primers in which the reverse-primer having the SEQ ID NO: the complementary strand base sequence of 17 is combined with 4 forward-primers having the above mentioned SEQ ID NOs: the base sequences of 10 to 13, and that of 3 primers in which the reverse-primer having the SEQ ID NO: the complementary strand base sequence of 18 is combined with 3 forward-primers having the above mentioned SEQ ID NOs: the base sequences of 14 to 16 so that 7 types in total were combined.

The PCR was performed using the commercially available enzyme system, the kit of AmpliTaq DNA polymerase (Takara Shuzo Co., Ltd.) in the following composition of reaction solution and the condition.

The overall amount of the reaction solution was adjusted to 50 µl by adding 1 µl each of the above described two primers having concentration of 50 pmol/pl, 5 µl of the reaction buffer attached to the enzyme, 5 µl of the dNTP-mixed solution attached to the enzyme, 10 ng of the DNA sample and further water. One unit of AmpliTaq DNA polymerase (Takara Shuzo) was added to this solution.

After the reaction solution was heated at 95° C and maintained for 5 min, the reaction condition was set as one cycle of being at 95° C for 20 sec, at 55° C for 30 sec and 72° C for 60 sec. The reaction of 30 cycles was performed under the above described condition, then the solution was further kept at 72° C for 5 min. After completion of the reaction, 2 µl was separately taken from 50 µl of the reaction solution, and the agarose gel electrophoresis and the ethidium bromide staining were performed to detect nucleic acid strands of the amplified products.

As a result, for the known 2 strains; E. coli JM109 and J1 (FERM BP-5352) not having the PHA synthesizing ability, no amplified products were detected at all. On the other hand, only for the PHA synthesizing microorganisms, one clear band was observed in any 7 types of PHA synthesizing microorganisms combined with the above described primers for the PCR as each amplified product. Herein, for the above described all 4 PHA synthesizing microorganisms, the fragment lengths of the PCR amplified products obtained from each of 7 types combined with the PCR primers correspond each other, which were shown in the following Table 2. In other words, since combination of 7 primers in total amplified the corresponding partial base sequences to each other from the PHA synthesizing enzyme genes originated in each of 4 PHA synthesizing microorganisms, the PCR-amplified products had almost the same amplified fragment length. From the above results, it was confirmed that any of 9 primers used in this example showed specificity applicable for detection of the PHA synthesizing microorganisms.

**Table 2**

| Base Sequence of Forward-Primer | Base Sequence of Reverse-Primer | PCR-Amplified Fragment Length |
|---|---|---|
| SEQ ID NO: 10 | complementary strand of SEQ ID NO: 17 | about 1.5 kbp |
| SEQ ID NO: 11 | complementary strand of SEQ ID NO: 17 | about 1.2 kbp |
| SEQ ID NO: 12 | complementary strand of SEQ ID NO: 17 | about 0.85 kbp |
| SEQ ID NO: 13 | complementary strand of SEQ ID NO: 17 | about 0.6 kbp |
| SEQ ID NO: 14 | complementary strand of SEQ ID NO: 18 | about 1.2 kbp |
| SEQ ID NO: 15 | complementary strand of SEQ ID NO: 18 | about 0.75 kbp |
| SEQ ID NO: 16 | complementary strand of SEQ ID NO: 18 | about 0.2 kbp |

### (Example 5) Detection Using Primers of PHA-Synthesizing Microorganisms (1)

As shown in example 4, the partial base sequences of the objective PHA synthesizing enzyme genes could be selectively amplified from the chromogenes of the PHA synthesizing microorganisms by the PCR method using the primers of the present invention to show the possible detection of the PHA synthesizing microorganisms. In the present example, when detecting the PHA synthesizing microorganisms, using primers treated with additional modification of the present invention, utilizing regions capable of coupling the labeled substance and solid phase carrier, and selecting only the objective PCR-amplified products, thereby one example attained with highly detectable sensitivity will be shown below.

Similarly in example 4, DNA was prepared from each of 6 bacteria. This DNA sample was submitted to the PCR using a commercially available enzyme system, AmpliTaq DNA polymerase (Takara Shuzo Co., Ltd.) in the following composition of reaction solution and the reaction condition.
2 primers prepared in example 2:
a biotinylated forward-primer of (SEQ ID NO: 10),
5'-Biotin-GCCTCGGAAAACACCTTGGGGCT-3' and a DNP-modified reverse-primer of (SEQ ID NO: complementary strand of 17),
5'-DNP-CAGCCACCAGGAGTCGGCGTGCTTG-3'

The overall amount of each reaction solution was adjusted to 50 µl by adding 1 µl each of the above described two primers having concentration of 20 pmol/µl, 5 µl of the reaction buffer attached to the enzyme, 2 µl of the dNTP-mixed solution attached to the enzyme, 10 pg each of YN2, H45, P91 and P161 strains as DNA samples, 10 ng each of other 2 bacteria and further water. One unit of AmpliTaq DNA polymerase (Takara Shuzo Co., Ltd.) was added to these reaction solutions.

After the reaction solution was heated at 95° C and maintained for 5 min, the reaction condition was set as one cycle of being at 95° C for 20 sec, at 55° C for 30 sec and 72° C for 60 sec. The reaction of 35 cycles was performed under the above described condition, then the solution was further kept at 72° C for 5 min. After completion of the reaction, the reaction mixture was submitted to spin column to remove the primer not to be reacted.

To the streptoavidin-fixed microplate, 100 µl of Tris-Cl buffer (pH 7.5) containing 0.15M NaCl and 0.05% Tween 20 was added beforehand and 10 µl of the above mixed solution in which the primer not to be reacted was removed was added to this. After allowing to stand at a room temperature for 30 min, the microplate was washed 3 times with 500 µl of the above Tris-Cl buffer. Based on this operation, the PCR-amplified products are fixed on the microplate by biotin originated in streptoavidin and the primer fixed on the surface of the microplate.

The alkaline phosphatase-labeled anti-DNP antibody was diluted 2000-fold with the above Tris-Cl buffer and 100 µl of that was added to the microplate after washing. After allowing to stand at a room temperature for 30 min, the microplate was washed 3 times with 500 µl of the above Tris-Cl buffer. Based on this operation, the alkaline phosphatase-labeled anti-DNP antibody is reacted (coupled) with a dinitrophenyl group (DNP) originated in the primer of the PCR-amplified products fixed on the surface of the microplate.

Then, 100 µl of the p-nitrophenyl phosphoric acid solution dissolved in 1M diethanol amine buffer is added onto the microplate in concentration of 4 mg/ml. In order to act the labeled enzyme, alkaline phosphatase on p-nitrophenyl phosphoric acid of the above substrate, using a microplate reader after allowing to stand at a room temperature for 30 min, the amount of enzymatic reaction products was measured in absorbance of 405 nm to be evaluated.

As a result, only in case of using DNA samples of 4 strains, YN2, H45, P91 and P161, the significant absorption could be observed compared with the background. In other words, by enzyme activity originated in the labeled enzyme, alkaline phosphatase, it was detectable that the objective PCR-amplified products having both biotin and the nitrophenyl group (DNP) originated in the primers were fixed on the microplate.

On the other hand, other two strains not showing the PHA synthesizing ability remained showing absorption around the background. Accordingly, as confirmed in example 4, the objective PCR-amplified products have not been obtained.

From the results of the present example, the primer concentration is reduced at 20 pmol/µl compared example 4, in addition, in the condition of substantial reduction of 10 ng to 10 pg for the amount of DNA samples, it is understood that sufficiently detectable PCR-amplified products are obtained by increasing the reaction cycle. Further, the selectivity made higher using the primer treated with additional modification and utilizing regions capable of coupling the labeled substance and solid phase carrier, consequently it was confirmed that sufficiently higher detection sensitivity was accomplished.

### (Example 6) Detection Using Primers of PHA-

### Synthesizing Microorganisms (2)

As shown in example 5, when detecting the PHA synthesizing microorganisms, using primers of the present invention treated with additional modification, utilizing regions capable of coupling the labeled substance and solid phase carrier, and selecting only the objective PCR-amplified products, thereby highly detectable sensitivity can be accomplished. As a result of the high detection sensitivity in this example, it was verified that a detection method for the PHA synthesizing microorganisms of the present invention was detectable also for the extremely small amount of the sample in the high degree of reliability, as shown in the following example.

Similarly in example 4, DNA was prepared from PHA synthesizing microorganisms, YN2, H45, P91 and P161 strains. This DNA sample was submitted to the PCR using a commercially available enzyme system, AmpliTaq DNA polymerase (Takara Shuzo Co., Ltd.) in the following composition of reaction solution and the reaction condition.

Two primers prepared in example 2:
a biotinylated forward-primer of (SEQ ID NO: 10),
5'-Biotin-GCCTCGGAAAACACCTTGGGGCT-3'
and a DNP-modified reverse-primer of (SEQ ID NO: complementary strand of 17),
5'-DNP-CAGCCACCAGGAGTCGGCGTGCTTG-3'.

The overall amount of each reaction solution was adjusted to 50 µl by adding 1 µl each of the above two primers having concentration of 20 pmol/pl, 5 µl of the reaction buffer attached to the enzyme, 2 µl of the dNTP-mixed solution attached to the enzyme, 10 pg, 1 pg, 100 fg and 10 fg of YN2, H45, P91 and P161 strains as DNA samples, respectively, and further water. For this each strain, one unit of AmpliTaq DNA polymerase (Takara Shuzo Co., Ltd.) was added to four reaction solutions each selected as the above four standards for the amounts of DNA samples.

After the reaction solution was heated at 95°C and maintained for 5 min, the reaction condition was set as one cycle of being at 95°C for 20 sec, at 55°C for 30 sec and 72°C for 60 sec. The reaction of 40 cycles was performed under the above described condition, then the solution was further kept at 72°C for 5 min. After completion of the reaction, the reaction mixture was submitted to spin column to remove the primer not to be reacted.

To the streptoavidin-fixed microplate, 100 µl of Tris-Cl buffer (pH 7.5) containing 0.15M NaCl and 0.05% Tween 20 was added beforehand and 10 µl of the above mixed solution in which the primer not to be reacted was removed was added to this. After allowing to stand at a room temperature for 30 min, the microplate was washed 3 times with 500 µl of the above Tris-Cl buffer. The PCR-amplified products are fixed on the microplate by this operation.

The alkaline phosphatase-labeled anti-DNP antibody was diluted 2000-fold with the above Tris-Cl buffer and 100 µl of that was added to the microplate after washing. After allowing to stand at a room temperature for 30 min, the microplate was washed 3 times with 500 µl of the above Tris-Cl buffer. By this operation, the alkaline phosphatase-labeled anti-DNP antibody is reacted (coupled) with the PCR-amplified products fixed on the microplate.

Then, 100 µl of the p-nitrophenyl phosphoric acid solution dissolved in 1M diethanol amine buffer is added onto the microplate in concentration of 4 mg/ml. In order to act the labeled enzyme, alkaline phosphatase on p-nitrophenyl phosphoric acid of the above substrate, using a microplate reader after allowing to stand at a room temperature for 30 min, the amount of enzymatic reaction products was measured in absorbance of 405 nm.

As a result, for 3 standards of 10 pg, 1 pg and 100 fg of the DNA sample amounts, the significant absorption could be observed comparing with the background. On the other hand, for the standard of 10 fg of the DNA sample amount, the absorption remained approximately not judging to be significant, compared with the background. The aforementioned results were the same as those of any PHA synthesizing microorganisms, YN2, H45, P91 and P161 strains.

As shown also in this example, the detection method for the PHA synthesizing microorganisms of the present invention was verified to be detectable for the PHA synthesizing microorganisms in the high degree of reliability even using an extremely small quantity of a sample.

### (Example 7) Detection Using Probes of PHA-Synthesizing Microorganisms (1)

Probes of the present invention were verified to show specificity applicable for detection of the PHA-synthesizing microorganisms. As the one example, by the dot plot method utilizing probes of the present invention, the example for detecting presence of the objective PHA synthesizing enzyme genes contained in DNAs of the PHA synthesizing microorganism genes will be shown as follows.

Similarly in example 4, DNAs were prepared from 6 bacteria in total being the known 2 strains; E. coli JM109 and J1 (FERM BP-5352) not having the PHA synthesizing ability and 4 strains; P. cichorii YN2 (FERM BP-7375), P. cichorii H45 (FERM BP-7374), P. putida P91 (FERM BP-7373) and P. jessenii P161 (FERM BP-7376) having the PHA synthesizing ability. After alkaline denaturation of each DNA sample, 1 µg each was blotted on nylon membrane (Tropilon-45, Tropix Inc.) using a dot blot apparatus (BRL). After drying at 80°C for 2 hr, the nylon membrane was placed in a vinyl bag and 3 ml of prehybridization solution (6×SSC, 5×Denhalt solution, 0.5% SDS, 100 µg/ml denatured salmon sperm DNA) was added to perform prehybridization at 60°C for 1 hr.

After 100 ng of a biotin-labeled oligonucleotide probe:
5'-GGCGAAAACAAGGTCAACGCCCTGACC-Biotin-3',
prepared in example 3 per 3 ml of the above prehybridization solution as a hybridization solution was added to the nylon membrane after thermal denaturation, hybridization was performed using 3 ml of this solution at 60°C for 2 hr. Thereafter, the nylon membrane was taken from the vinyl bag and washed 3 times with 6×SSC and 0.5% SDS solution at 60°C for each 5 min.

Detection of DNA treated with hybridization of the aforementioned biotin-labeled probe was performed utilizing the chemoluminescence method by labeled enzyme, alkaline phosphatase and AMPPD with alkaline phosphatase-labeled streptoavidin coupled with biotin of the probe, using Southern Light (Tropix Inc.) according to the attached protocol.

As a result, for blotting DNAs originating in PHA synthesizing microorganisms, YN2, H45, P91 and P161 strains, the extremely strong positive reaction could be observed. On the other hand, for other 2 strains not having the PHA synthesizing ability, the positive reaction could not be detected. Accordingly, it was confirmed that the probes of the present invention used for the present example showed specificity applicable for detection of the PHA synthesizing microorganisms.

### (Example 8) Detection Using Probes of PHA-Synthesizing Microorganisms (2)

As shown in example 7, the probes of the present invention show specificity applicable for detection of the PHA synthesizing microorganisms such as YN2, H45, P91 and P161 strains, and it was verified that the present example could be applied to various hybridization methods, as shown in the following example.

6 bacteria in total being the known 2 strains; E. coli JM109 and J1 (FERM BP-5352) not having the PHA synthesizing ability and 4 strains; P. cichorii YN2 (FERM BP-7375), P. cichorii H45 (FERM BP-7374), P. putida P91 (FERM BP-7373) and P. jessenii P161 (FERM BP-7376) having the PHA synthesizing ability were cultured by the conventional method, respectively. The cultured bacteria were collected, then after washing with 0.1 M sodium phosphate buffer (pH 8.0), using the above described buffer, the cell suspensions were prepared so as to be set as 2×10⁷ cells/ml for each cell count.

50 µl of 6% formaldehyde solution was added to 50 µl of the prepared cell suspension to fix the bacteria. Then, 30 µl of the fixed cell suspension was dropped onto a slide glass coated with 0.1% gelatin and 0.01% chrome alum, and dried in air. This slide glass fixed with the bacteria sample was soaked in 90% methanol and 3% formaldehyde solution for 10 min to fix the bacteria again, then washed with pure water.

The slide glass fixed with the fixed bacteria sample performed with the above treatment was soaked in 10 mM Tris-Cl buffer (pH 8.0) containing 50 mM NaBH₄ at room temperature for 30 min in a shading state. Thereafter, it was washed with pure water and dried in air.

The probe in which FITC (Fluorescein isothiocyanate)-labeled streptoavidin was coupled beforehand with biotin-labeled oligonucleotide probe:
5'-GGCGAAAACAAGGTCAACGCCCTGACC-Biotin-3', prepared in example 3, was used. A solution of 30 µl in which this FITC-labeled probe in concentration of 5 ng/µl was added to the hybridization solution (0.1 M Tris-Cl buffer (pH 8.0), 0.75M NaCl, 5 mM EDTA, 10% dextran sulfate, 0.2% BSA (Bovine Serum Albumin) and 0.01% polyadenylic acid) was dropped onto a slide glass. The slide glass was placed in an airtight container and the reaction was carried out at 45°C for 1 hr in a shading state.

After the reaction, the slide glass was washed with the SET buffer (Tris-Cl buffer (pH 8.0), 0.2 mM EDTA and 30 mM NaCl) and dried in air in a shading state. To detect the bacteria hybridized with the FITC-labeled probe, the presence or absence of fluorescence was investigated performing the microscopic examination by the epi-illumination type fluorescence microscope of Olympus. Mercury lamp was used for an excitation source to be observed by the B excitation. As the results of the microscopic examination, the fluorescence was observed in any of the PHA synthesizing microorganisms, YN2, H45, P91 and P161 strains. On the other hand, no fluorescence could be observed in other two bacteria not having the PHA synthesizing ability.

As shown in the present example, of course, in case of using DNA samples prepared from microorganisms and also in the other procedure for using intact bacteria as a sample, it was confirmed that the probes of the present invention had sufficiently specificity applicable for detection of the objective PHA synthesizing microorganisms.

## Claims

1. A nucleic acid fragment consisting of a base sequence shown in SEQ ID No. 5, or complementary base sequence thereof, or modified sequence thereof formed by a partial deletion of the base sequence or complementary base sequence, wherein the modified sequence is capable of hybridizing to a polyhydroxyalkanoate (PHA) synthetase gene from Pseudomonas cichorii YN2 (FERM P-17411), P. cichorii H45 (FERM P-17410), P. putida P91 (FERM P-17409) and P. jessenii P161 (FERM P-17445) under hybridization conditions of 6×SSC, 5× Denhart solution, 0.5% SDS, 100 µg/ml denatured salmon sperm DNA at 60°C for 2 hours, and washing conditions of 6xSSC and 0.5% SDS solution at 60°C three times for each 5 minutes.

2. A primer or probe having 10 to 50 nucleotides in length and comprising a nucleic acid fragment according to claim 1.

3. A combined primer or probe, comprising
a reverse primer being the complementary base sequence of SEQ No 5; and
a forward primer being a sequence selected from any of the sequences shown in SEQ ID Nos. 1 to 4.

4. A primer or probe as defined in claim 2 or a combined primer or probe as defined in claim 3, in which, as an additional modification, a marker bound onto a molecule of said primer or probe, and/or a moiety capable of binding to a solid-phase carrier is introduced.

5. A primer composition comprising a combination of two kinds of nucleic acid fragments each having 10 to 50 nucleotides in length with a substantial difference in base sequence, wherein at least one of said two kinds of nucleic acid fragments is a primer according to claim 2, and a marker, and/or a moiety capable of binding to a solid-phase carrier may be introduced into each molecule of said two nucleic acid fragments.

6. The primer or probe according to claim 2 or the primer composition according to claim 5, wherein said primer or probe comprises at least one kind of nucleic acid fragment subjected to an additional modification, and the additional modification in one kind of said nucleic acid fragment is introduction of a marker or moiety capable of binding to a solid-phase carrier into a 5'-terminal side of the nucleic acid fragment.

7. The primer or probe or the primer composition according to claim 6, wherein a marker or a moiety capable of binding to a solid-phase carrier to be introduced into a molecule as an additional modification is any of biotin residue, 2,4-dinitrophenyl group, and digoxigenin residue.

8. A method of detecting a PHA synthesizing microorganism, wherein said method uses at least one primer, probe, combined primer or probe, or primer composition according to any one of claim 2 to 7.

9. The method according to claim 8, used for the detection of at least one microorganism selected from the group consisting of Pseudomonas cichorii YN2 (FERM P-17411), P. cichorii H45 (FERM P-17410), P. putida P91 (FERM P-17409) and P. jessenii P161 (FERM P-17445).

## Patentansprüche

1. Nucleinsäurefragment, bestehend aus einer Basensequenz, die mit der SEQ-ID-Nummer 5 dargestellt wird, oder einer komplementären Basensequenz davon oder einer modifizierten Sequenz davon, gebildet durch eine teilweise Deletion der Basensequenz oder der komplementären Basensequenz, wobei die modifizierte Sequenz zu einer Hybridisierung in der Lage ist, mit einem Polyhydroxyalkanoat(PHA)-Synthetase-Gen von Pseudomonas cichorii YN2 (FERM P-17411), P. cichorii H45 (FERM P-17410), P. putida P91 (FERM P-17409) und P. jessenii P161 (FERM P-17445) unter Hybridisationsbedingungen in Form von 6 x SSC, 5 x Denhartlösung, 0,5 % SDS, 100 µg/ml denaturierte Lachsspermium-DNA bei 60°C für 2 Stunden und Waschbedingungen in Form von 6 x SSC und 0,5 %ige SDS-Lösung bei 60°C dreimal für jeweils 5 Minuten.

2. Primer oder Sonde mit einer Länge von 10 bis 50 Nucleotiden und ein Nucleinsäurefragment nach Anspruch 1 umfassend.

3. Kombinierter Primer oder kombinierte Sonde, umfassend:
einen Umkehrprimer, der die komplementäre Basensequenz von SEQ-Nummer 5 ist; und
einen Vorwärtsprimer, der eine Sequenz ist, welche aus irgendeiner der bei SEQ-ID-Nummern 1 bis 4 dargestellten Sequenzen ausgewählt ist.

4. Primer oder Sonde nach Anspruch 2, oder kombinierter Primer oder kombinierte Sonde nach Anspruch 3,
wobei als zusätzliche Modifizierung eine an ein Molekül des Primers oder der Sonde gebundene Markierung und/oder eine Einheit (engl. "moiety"), die sich an einen Festphasenträger binden kann, eingeführt ist.

5. Primerzusammensetzung, umfassend: eine Kombination von zwei Arten von Nucleinsäurefragmenten mit einer Länge von je 10 bis 50 Nucleotiden mit einem wesentlichen Unterschied bei der Basensequenz, wobei zumindest eine dieser beiden Arten von Nucleinsäurefragmenten ein Primer gemäß Anspruch 2 ist, und eine Markierung und/oder eine Einheit, die sich an einen Festphasenträger binden kann, in jedes Molekül dieser beiden Nucleinsäurefragmente eingeführt sein kann.

6. Primer oder Sonde nach Anspruch 2 oder Primerzusammensetzung nach Anspruch 5,
wobei der Primer oder die Sonde zumindest eine Art eines Nucleinsäurefragmentes umfasst, welches einer zusätzlichen Modifizierung unterzogen wurde, und die zusätzliche Modifizierung bei einer Art des Nucleinsäurefragmentes vorliegt als Einführung einer Markierung oder Einheit, die sich an einen Festphasenträger binden können, in die 5'-Endseite des Nucleinsäurefragments.

7. Primer oder Sonde oder Primerzusammensetzung nach Anspruch 6,
wobei eine Markierung oder eine Einheit, die sich an einen Festphasenträger binden können, als zusätzliche Modifizierung in ein Molekül einzuführen ist, vorliegt als irgendein Rest der Gruppe, bestehend aus einem Biotinrest, einer 2,4-Dinitrophenylgruppe und einem Digoxigenin-Rest.

8. Verfahren zum Nachweis eines PHA-synthetisierenden Mikroorganismus,
wobei zumindest eines von den Materialien, nämlich Primer, Sonde, kombinierter Primer oder kombinierte Sonde oder Primerzusammensetzung nach einem der Ansprüche 2 bis 7, verwendet wird.

9. Verfahren nach Anspruch 8,
zur Verwendung für den Nachweis von zumindest einem Mikroorganismus, der ausgewählt ist aus der Gruppe, bestehend aus Pseudomonas cichorii YN2 (FERM P-17411), P. cichorii H45 (FERM P-17410), P. putida P91 (FERM P-17409) und P. jessenii P161 (FERM P-17445).

## Revendications

1. Fragment d'acide nucléique consistant en une séquence de bases représentée dans la SEQ ID N° 5, ou une séquence de bases complémentaire de celle-ci, ou une séquence modifiée de celle-ci formée par une délétion partielle de la séquence de bases ou séquence de bases complémentaire, dans lequel la séquence modifiée est capable de s'hybrider à un gène de polyhydroxyalcanoate (PHA)-synthétase provenant de Pseudomonas cichorii YN2 (FERM P-17411), P. cichorii H45 (FERM P-17410), P. putida P91 (FERM P-17409) et P. jessenii P161 (FERM P-17445) dans des conditions d'hybridation consistant en SSC 6x, solution de Denhart 5x, 0,5 % de SDS, 100 µg/ml d'ADN dénaturé de sperme de saumon à 60°C pendant 2 heures, et conditions de lavage consistant en SSC 6x et solution à 0,5 % de SDS à 60°C trois fois pendant 5 minutes à chaque fois.

2. Amorce ou sonde ayant une longueur de 10 à 50 nucléotides et comprenant un fragment d'acide nucléique suivant la revendication 1.

3. Amorce ou sonde combinée, comprenant
une amorce inverse consistant en la séquence de bases complémentaire de la SEQ ID N° 5 ; et
une amorce directe consistant en une séquence choisie parmi n'importe lesquelles des séquences représentées dans les SEQ ID N° 1 à 4.

4. Amorce ou sonde suivant la revendication 2 ou amorce ou sonde combinée suivant la revendication 3, dans laquelle, comme modification supplémentaire, un marqueur lié à une molécule de ladite amorce ou sonde et/ou un groupement capable de se lier à un support en phase solide est introduit.

5. Composition d'amorce comprenant une association de deux types de fragments d'acide nucléique ayant chacun une longueur de 10 à 50 nucléotides présentant une différence substantielle dans la séquence de bases, dans laquelle au moins un desdits deux types de fragments d'acide nucléique est une amorce suivant la revendication 2, et un marqueur, et/ou un groupement capable de se lier à un support en phase solide peut être introduit dans chaque molécule desdits deux fragments d'acide nucléique.

6. Amorce ou sonde suivant la revendication 2 ou composition d'amorce suivant la revendication 5, dans laquelle ladite amorce ou sonde comprend au moins un type de fragment d'acide nucléique soumis à une modification supplémentaire, et la modification supplémentaire dans un type dudit fragment d'acide nucléique est l'introduction d'un marqueur ou groupement capable de se lier à un support en phase solide du côté 5'-terminal du fragment d'acide nucléique.

7. Amorce ou sonde ou composition d'amorce suivant la revendication 6, dans laquelle un marqueur ou un groupement capable de se lier à un support en phase solide à introduire dans une molécule comme modification supplémentaire est n'importe laquelle des entités consistant en un résidu biotine, un groupe 2,4-dinitrophényle et un résidu digoxigénine.

8. Méthode de détection d'un micro-organisme synthétisant des PHA, ladite méthode utilisant au moins une amorce, sonde, amorce ou sonde combinée, ou composition d'amorce suivant l'une quelconque des revendications 2 à 7.

9. Méthode suivant la revendication 8, utilisée pour la détection d'au moins un micro-organisme choisi dans le groupe consistant en Pseudomonas cichorii YN2 (FERM P-17411), P. cichorii H45 (FERM P-17410), P. putida P91 (FERM P-17409) et P. jessenii P161 (FERM P-17445).
